# EUROPEAN PATENT SPECIFICATION

(11) **EP 2 001 514 B1**
(45) Date of publication and mention of the grant of the patent: **31.05.2017**
(21) Application number: 06745236.7
(22) Date of filing: 17.03.2006
(51) Int. Cl.: A61K 47/60, B01J 13/16, B01J 13/00, B82Y 5/00

(54) **PREPARATION OF MICRO OR NANO SYSTEMS BASED ON INORGANIC OXIDES WITH CONTROLLED POROSITY FOR CARRYING BIOLOGICALLY ACTIVE SUBSTANCES**
HERSTELLUNG VON MIKRO- ODER NANOSYSTEMEN AUF BASIS VON ANORGANISCHEN OXIDEN MIT KONTROLLIERTER POROSITÄT ZUM TRAGEN VON BIOLOGISCH WIRKSAMEN SUBSTANZEN
PRÉPARATION DE MICROSYSTÈMES OU DE NANOSYSTÈMES À BASE D'OXYDES INORGANIQUES AYANT UNE POROSITÉ CONTRÔLÉE SERVANT À SUPPORTER DES SUBSTANCES BIOLOGIQUEMENT ACTIVES

(43) Date of publication of application: 17.12.2008
(73) Proprietor: NANOSILICAL DEVICES SRL, 87036 Rende (IT)
(72) Inventor: AIELLO, Rosario, 87036 Rende (CS) (IT); MAIONE, Umberto, 87100 Cosenza (IT); PASQUA, Luigi, 87100 Cosenza (IT); TESTA, Flaviano, 87040 Marano Marchesato (CS) (IT)
(74) Representative: Perrotta, Aldo
(86) International application number: PCT/IT2006/000167
(87) International publication number: WO 2007/108016

(56) References cited:
- EP-A- 1 358 873
- WO-A-99/12640
- WO-A-2005/110592
- TOURNÉ-PÉTEILH C. ET AL.: "Synthesis and characterization of ibuprofen anchored MCM-41 silica and silica gel" NEW J. CHEM., vol. 27, 2003, pages 1415-1418, XP007901732 cited in the application
- RAMILA A ET AL.: "Mesoporous MCM-41 as Drug Host System" JOURNAL OF SOL-GEL SCIENCE AND TECHNIOLOGY, vol. 26, 2003, pages 1109-1202, XP008075018 cited in the application
- MUNOZ B ET AL: "MCM-41 Organic Modification as drug delivery rate regulator" CHEMISTRY OF MATERIALS, AMERICAN CHEMICAL SOCIETY, WASHINGTON, US, vol. 15, 2003, pages 500-503, XP002251472 ISSN: 0897-4756 cited in the application
- SCHACHT S ET AL: "OIL-WATER INTERFACE TEMPLATING OF MESOPOROUS MACROSCALE STRUCTURES" SCIENCE, AMERICAN ASSOCIATION FOR THE ADVANCEMENT OF SCIENCE,, US, vol. 273, 9 August 1996 (1996-08-09), pages 768-771, XP002923904 ISSN: 0036-8075 cited in the application
- VALLET-REGI M ET AL: "A NEW PROPERTY OF MCM-41: DRUG DELIVERY SYSTEM" CHEMISTRY OF MATERIALS, AMERICAN CHEMICAL SOCIETY, WASHINGTON, US, vol. 13, no. 2, 1 February 2001 (2001-02-01), pages 308-311, XP001005172 ISSN: 0897-4756 cited in the application
- Mats Silvander ET AL: "Linkage identity is a major factor in determining the effect of PEG-ylated surfactants on permeability of phosphatidylcholine liposomes", Chemistry and Physics of Lipids, vol. 126, no. 1, 1 November 2003 (2003-11-01), pages 77-83, XP055179707, ISSN: 0009-3084, DOI: 10.1016/S0009-3084(03)00094-X
- MOHAMED M Z ET AL: "SYNTHESIS AND EVALUATION OF NEW AMPHIPHILIC POLYETHYLENE GLYCOL-BASED TRIBLOCK COPOLYMER SURFACTANTS", JOURNAL OF SURFACTANTS AND DETERGENTS, SPRINGER, BERLIN, DE, vol. 8, no. 2, 1 April 2005 (2005-04-01), pages 175-180, XP001228389, ISSN: 1097-3958, DOI: 10.1007/S11743-005-344-4

## Description

### FIELD OF THE INVENTION

The invention is related to a process for the preparation of a multifunctional system which uses an inorganic oxide with controlled porosity carrying an active biological species (drug) and, at the same time, supports on the external surface a targeting function with biological activity able, in particular, to be recognized by particular biological tissues.

### BACKGROUND OF INVENTION

In the last years MOBIL researchers introduced the family of mesoporous molecular sieves named M41S. The phase named MCM-41 is the member of the family M41S characterized by regular hexagonal array of mesopores with uniform diameter. They are synthesized using cationic surfactants as structuring agents (C. T. Kresge, M.E, Leonowicz, W.J. Roth, J. C Vartuli, J. S. Beck, Nature ,359, (1992), 710-712; J.S. Beck, J.C. Vartuli, V.J. Roth, M.E. Leonowicz, C.T. Kresge, K.D. Schmitt, C.T.W. Chu, D.H. Olson, E.W. Sheppard, S.B. Mc Cullen, J.B. Higgins and J.L. Schlenker, J. Am. Chem. Soc. 114 (1992) 10834). Mesoporous materials have been obtained using anionic surfactants (Q. Huo, D.I. Margolese, U. Cielsa, P. Feng, T.E. Gier, P. Sieger, R. Leon, P.M. Petroff, F. Schüth, G.D. Stucky, Nature, 1994, 368, 317) and neutral surfactants such as alchilamine with long chain [P.T. Tanev, T.J. Pinnavaia, Science, 1995, 267, 865] and polyethilene oxides [G. Attard, J.C. Glyde, C.G. Göltner, Nature, 1995, 378, 366; S.A. Bagshaw, E. Prouzet and T.J. Pinnavaia, Science, 1995, 269, 1242; D. Zhao, J. feng, Q. Huo, N. Melosh, G.H. Fredrickson, B.F. Chmelka, G.D. Stucky, Science, 1998, 279, 548; D. Zhao, Q. Hou, J. Feng, B.F. Chmelka, G.D. Stucky, J. Am. Chem. Soc., 1998, 120, 6024]. The diameter of mesopores can be tailored from approximatively 20 A to ca. 100 A depending on the surfactant and the additives used in the synthesis. The aluminosilicate-type MCM-41 have been considered since their discovery as the natural extension of zeolites and immediately fields of applications such as catalysis or separation processes of substrates sterically hindered too large to diffuse in the narrow zeolites micropores have been defined. The discovery of ordered mesoporous silica opened the route for new developments , for example in the "host-guest" synthesis of nanostructured materials. Active sites with high definition has been created by grafting of metallocene complexes on mesoporous silicas. The synthesis strategies offered by the chemistry of sol-gel processes together with the ability of surfactants to self-assembly liquid-crystalline mesophases represents a modular code which allows to develop innovative materials with complex architectures which makes the ideal substrate in the design of a structured nanofunctional material. The hybridization of a inorganic precursor allows, in fact, the design and the development of innovative multifunctional materials with complex structure and with various properties. Hybrid organic-inorganic materials have been obtained by covalently binding chemically active groups to the inorganic structure of the mesoporous materials through post-synthesis grafting or through simultaneous condensation of silossanic and organo-silossanic reactants, the latter being provided with a not hydrolizable Si-C bond.

The synthesis of hydrophobic mesoporous materials for simultaneous removal of the structuring surfactant and modification of the external surface has been obtained treating the as-made MCM-41 material with trialchylclorosilanes. Recently, the preparation of mesoporous silicates and aluminosilicates with different synthesis methods and their chemical modification of the surface has been reported. Typical pore volume of the activated materials with regular porosity can be utilized in order to immobilize molecular guest with very different kind or structure. Among different possible applications those regarding biologically active molecules, with particular attention to the drug controlled release, seem to be very promising.

Enzymes and proteins can be adsorbed on the hydroxilated surface of activated inorganic oxides or on the functionalized pore walls of hybrid materials with regular porosity. MCM-41 mesoporous silicas have been studied for the immobilization of non-steroidal anti-inflammatory drugs provided of a carboxilic acid functional group. The confinement in the matrix can be obtained through phisisorption of the molecule or through chemical grafting, for example, on the suitably functionalized silica surface. [M. Vallet.Regi, A. Ramila, R.P. Del Real, and J. Perez pariente, Chem. Matter., 2001, (13), 308; G.Cavallaro, P. Pierro F.S. Palumbo, F. Testa, L. Pasqua, R. Aiello Drug Delivery 11, 41-46, 2004*;* B. Munoz, A. Ramila, J. Perez Pariente, I. Diaz, and M. Vallet Regi, Chem. Mater., 2003, 15 500; A. Ramila, B. Munoz, J. Perez Pariente, M. Vallet-Regi, J. Sol-Gel Sci. Technol., 26 (2003) 1199; C. Tourné-Péteihl, D. Brunel, S. Bégu, B. Chiche, F. Fajula, D.A. Lerner J.M. Devoisselle, New. J. Chem., 27 (2003) 1415]. In the latter case [New. J. Chem., 27 (2003) 1415] ibuprofen drug has been covalently linked to the surface of MCM-41 type mesoporous silica. The esterification has been realized using the ibuprofen carboxilic group in the opening of the epoxidic ring of 3-glycidoxypropylsilane grafted on the silica surface. A similar system based on a pro-drug involves the advantage due to the enzimatic activation through the breakage of the ester bond due to esterasi "in vivo".

Pentagastrine peptide, an activator of the gastric secretion, has been introduced through soaking from the solution in a mesoporous silica synthesized using a Tween-80 type surfactant [C. Tourné-Péteihl, D.A. Lerner, C. Charnay, L. Nicole, S. Bégu, J.M. Devoisselle, ChemPhysChem, 3 (2003) 281]. Recently, a system for the controlled drug release with nanospheres of MCM-41 type mesoporous silica has been optimized. Chemically removable caps made of nanocrystals of cadmium sulphide in order to hold different drugs and neurotransmitters inside the Mesoporous framework have been utilized [C. Lai, B.G. Trewyn, D.M. Jeftinija, K. Jeftinija, S. Xu, S. Jeftinija, V.S. Lin, J. Am. Chem. Soc., 125 (2003) 4451].

The use of materials and systems at the nanometer scale is in fast development only since few years. Nanotechnological approaches devoted, not only to the controlled drug release but also to the molecular imaging and use as biomarkers or biosensors, have been recently proposed. Different experiments concerned face the use of silica based materials. In 1999 silica glasses which incorporate biological molecules as systems for drug controlled release have been patented [U.S. Patent, n. 5.874.109, 1999]. Therasphere® system is a new therapeutic system, commercialized by MDS Nordion, utilized as local radiotherapic agent in the hepatic carcinome. It is made of unsoluble glass microspheres partially constituted by a β emitter introduced through the hepatic artery.

The active targeting of a drug on a characteristic tissue can be based on the recognition of a functional group linked to a preselected molecule.

Folic acid can be employed in the active targeting of a drug. Receptors of folic acid, in fact, constitute a useful target in the active carrying of antitumoral drugs.
1) In fact, they are overregulated in many typologies of human cancer including malignant tumors, colorectal, ovary, brain, kidney, breast, lung, mieloids cells, allowing to neoplastics cells, in case of limited availability, a competitive supply.
2) The access to folate receptor in the normal tissues which express it is seriously restricted by its position on the external face of the apical membrane of the polarized epitelia.
3) Density of folate receptors seems to increase with the enhancement of the disease. Although the transport mechanism of the folic acid in the cell by the folate receptor is not clearly known. it is evident that the folate conjugated are assumed by mammals cells through an endocitosis process mediated by the receptor. Endocitosis mechanisms involves particles smaller than 100 nm. Successively to the conjugation to the folate receptor on the surface of the tumoral cell, folate conjugate independently of the size penetrate and is driven to intracellular compartments called endosomes. Endosomes containing folate conjugated present a pH value ca. 5 due to a process called endosomes acidification which is responsible for the dissociation between conjugate and receptors [Y. Lu, P.S.Low, Adv. Drug. Deliv. Rev., 54 (2002) 675].

Targeting of folate receptors can be employed for the intracellular release of macromolecular therapeutical agents [K.Kono, M.Liu, J.M.J. Frechèt, Bioconjugate Chem., 1999, 10, 1115] but also drugs that do not need intracellular release can be carried to neoplastic tissues. In this way tumors of difficult therapeutic treatment with classical methods can be specifically reached using conjugated therapeutic agents-folic acid.

With the objective of exploiting the characteristics of the overexpression of the folic acid receptor this one has been conjugated to both low molecular weight drugs and to macromolecular complexes as molecular targeting agent of molecules linked to neoplastic cells. Polyether-based systems with folate residues on the external surface as active drug carriers with specific potentiality between tumoral cells, have been prepared [K.Kono, M.Liu, J.M.J. Frechèt, Bioconjugate Chem., 1999, 10, 1115].

A nanodevice for drug release which allows an intracellular release and, at the same time, is provided with imaging properties has been obtained by covalently bonding fluorescein, methotrexate and folic acid, to the surface of a ethylendiaminic nucleus of a polyamidoamine-based dendrimer [A. Quintana, E. Raczka, L. Piehler,I. Lee, A. Myc, I. Mayoros, A.K. Patri, T. Thomas, J. Mulè, J.R. Baker Jr, Pharm. Res. 19 (2002) 1310]. Recently, a conjugated folate-ciclodestrine has been prepared and characterized [P. Caliceti, S. Salmaso, A. Semenzato, T. Carofiglio, R. Fornasier, M. Fermeglia, M. Ferrone, and S. Pricl, Bioconjugate Chem., 2003, 14, 899]. The conjugation of folic acid to the macromolecules produced systems that showed drug release "in vitro" on tumorals cells which express folic acid receptors in almost all the situations. Macromolecular targeting mediated by folic acid produced only partial results "in vivo" because of the problem-connected with the penetration of solid tumors by the macromolecules. Anyway, examples where the folate carrying has significantly improved results of a therapy based on macromolecular vectors, leading in many cases to a complete recovery.
- In the document FR 2794115 a silica sol made of nanoparticles with average size between 3 and 50 nm has been treated on its surface by grafting or adsorbing an organic molecule or by doping with a dopant substance. These substances have been monodispersed in a liquid phase and show a concentration higher or equal to 10 %.
- In the document USP 6548264 a new method for the preparation of nanoparticles whose core is wrapped in a silica shell is claimed. Nanoparticles have been prepared by precipitation starting from reactants dissolved in the aqueous medium of a water-in-oil emulsion. A reactive silicate has been added in order to coat the nanoemulsions core. Coated silica particles have been functionalized using different methods with the objective of using them, as an example, as dye-doped, metallic, semiconductor, magnetic and drug-containing particles.

Nanoparticles core which have an average diameter lower than 1 micron and preferably ranging between 1 and 100 nm or 2 and 10 nm can be magnetic and can include a metal chosen between magnetite, maeghmite and greigite. In other cases the core can include a pigment that can be potassium permanganate, potassium dichromate, nickel sulphate, cobalt chloride, iron (III) chloride and cuprum nitrate. In the same way it can include a dye as Ru/Bpy, Eu/Bpy and similar or a metal as Ag or Cd. Silica shell which coats nanoparticles can be derivatized with a functional group as a protein (e.g. an antibody), a nucleic acid (e.g. an oligonucleotide) biotine or streptavidine. Finally, the proposed method in the invention can be used to produce silica-coated nanoparticles derivatized with proteins which contain a metal such as magnetite, maeghmite or greigite. In another aspect the invention features a method for identifying cells expressing preselected molecule. In this case a cells plurality in which at least a part of them express the particular molecule is mixed with a plurality of silica-coated nanoparticles in conditions allowing the nanoparticles to specifically bind the cells expressing the preselected molecule. In another embodyment the silica-coated nanoparticles are fluorescent. The nanoparticle is solid and preferably without pores but for some applications can be made porous by degrading the nanoparticles coating with a corrosive agent and eventually by reconstructing it with silica. In general solid particles are preferred when it is desired to isolate them from the outside environment while those porous are preferred when it is desired to increase the surface area of the coating in contact with the outside environment. The core can be formed by any substance compatible with the coating with the goal to satisfy to particular requirements for the application for the use of the particle. As an example, when the particle needs to be magnetic the core is made up of a magnetic metal and the particles can be used in applications such as cells separation/purification or diagnostic imaging. The core can be also made of a mixture of different substances. As an example, in a magnetic and dye-doped particle, the core can be constituted by a magnetic metal and a inorganic salt useful as pigment. The core can have any size but it must be lower than that of the desired particle. For certain applications the core must preferably have a diameter ranging between 1 and ca. 200 nm. As an example, because particles with size lower than 100 nm can be excreted by animals while those with higher diameter can be retained especially in the liver and in the spleen, in the case where diagnostic and therapeutic applications are preferred and, consequently, is recommended that the particles are not retained, the core must be small enough so to be contained in a particle with size lower than 100 nm. Coating can be made with a polymer (polystyrene, polyvinylchloride, acrylic polymers), a polysaccharide as destrane, an inorganic oxide as alumina, silica, or a mixture of them. In many applications silica is preferred because it is relatively inert in many environments, is biocompatible, prevents agglomeration with others nanoparticles in a dispersion and can be easily derivatized with many functional groups. Coating can be made by a layer that coats the core and by a second layer that coats the first one. This second layer can be made of a biodegradable material, for example a sugar or a polymer, impregnated with a drug. This biodegradable material, when introduced in a animal organism, can be dissolved and the drug can gradually diffuse. Coating can be made by 3, 4, 5 or more separated layers. Functional groups can be derivatized on the coating. They can be any chemical or biological function that can be linked to the nanoparticle through the coating: as an example, one or more proteins such as antibodies (monoclonals, polyclonals), enzymes, biotine, streptavidine, nucleic acids molecules, chemosensors, fluorescent probes and biochemical groups as amines and carboxylate groups.

Document USP 6548264 describes a system not based on a oxide with ordered porosity but a nanoparticles general typology made of a silica or any other material shell which contains a core of different nature and composition. Porosity is eventually obtained in the shell by chemical methods. When the functionalization is desired on the shell, it is finalized to the specific interaction based on the recognition of a characteristic species and to separation. When the systems are impregnated with a drug, they release it after the erosion of a further erodible layer created on the shell which is eventually and previously made porous.

Document US 6319715 claims a methodology to improve the release of nucleic acid molecules to "in vitro" cells through formation of a complex nucleic acid-transfecting agent-nanoparticle. The method foresees the incubation of silica nanoparticles with the nucleic acid. Nucleic acid-nanoparticles complexes are allowed to sediment on to the cells. Release of genetic material on the cells is carried out through standard transfection agents. Essential requirement of the nanoparticles is that they are non toxic and biocompatible, able to associate with nucleic acids or with complexes of nucleic acids and can sediment in an aqueous medium in order to increase the nucleic acid concentration on the cells surface. It is preferable that they do not form aggregates.
- Document US 6319715 does not foresee the use of inorganic oxides with controlled porosity and high pore volume where the therapeutic agent can be introduced. Furthermore, the cited document describes a passive release in which the closeness between particle and cell is induced by a sedimentation process without use of any directioning function.
- Document US 2003/203206 describes a preparation methodology of hexagonal mesoporous silica able to produce a controlled release of an incorporated substance. It is provided of a photo-dimerizable organic functional group at the pore opening which lock its entrance. The incorporated active substance can be released where and when desired after a photoinduced splitting of the dimer.

Document US 2003/203206 describes a system for the carrying of biologically active substances in a mesoporous silica matrix provided with a system for the diffusion control of the same substances but not able to target characteristic tissues through a recognition mechanism.
- Document WO01/12846 claims a method for the preparation of support loaded with biomolecules constituted by the phase of: a) activation of the support surface through a silane carrying an amminic group; b) loading of the activated surface of the support with biomolecules; c) treatment of the loaded support with a acid, basic or neutral solution. The method can be, for example, utilized in the preparation of serial analytical probes. In this method the aminic groups present after the loading of the biomolecule (which could generate undesirable interactions in the medium where the loaded support is used) are selectively removed from the surface without affecting the loaded part of the surface. The method of the invention is applicable in all the situations in which free aminic groups must be selectively removed from the surface of the metallic oxide loaded with biomolecules.

The pH of the solution where the loaded support can be treated, substantially depends on the kind of the molecule linked to the surface and on the bond (covalent or adsorption). Supports prepared through the method of the invention are very useful for diagnostic tools as, for example, in the analytical probes. For example, in this regard are very suitable series or microseries without free aminic groups. The series can include different biomolecules in different points allowing the detection of multiple analytes. Generally an analyte is a substance, normally a biomolecule, which detection is possible because of its ability to specifically interact with a certain reactant. A sample that includes an analyte is put in contact with loaded support prepared through the method described in the invention. The analyte is allowed to react with a biomolecule which is linked to the surface of the support. The interaction can be followed through a detection method.

Document WO 01/12846 describes a system based on a surface where biomolecules or different kind of biomolecules in different "spots" able to recognize other species in a mixture are grafted or adsorbed. Moreover, it does not foresee transport of any therapeutic agent and the recognition is finalized to the detection.

### Detailed Description

The invention has been realized starting from inorganic oxides with regular porosity. The porosity control is carried out during the synthesis through the use of whatever surfactant or through a mechanism of molecular imprinting. Oxides can be provided of a function introduced during the synthesis of the material or through a post-synthesis grafting process. The introduced function is useful for the grafting of molecules or particles different for nature and size on the external surface of the mesoporous material. Where not specified such a molecule or particle will be generically named as **targeting function.** Among the possible conjugation of the external surface, particularly important seems to be the use of biologically active or involved in cellular recognition molecules, peptides or antobodies. The functionalization is addessed only to the external surface using the as-made material before the extraction processes which make the internal pores accessible to the reactants.

The introduction of the targeting function occurs before the surfactant removal and the immobilization of the active substance.

The grafting of the targeting function drastically reduces the operative conditions successively needed for the surfactant removal. In fact, they must be compatible with the chemical stability and the biological activity of the introduced group. Pore volume available after the extraction processes is useful for the drug-loading and the transport of biologically active substances for their release on the target site. Molecules, atoms, ions having very different kind and sizes such as antitumoral drugs, anti-inflammatory, hormones, peptides, DNA fragments, can be carried. Where not specified the molecule, ion, or material will be generically indicated with the term **active substance.**

The approach to the synthesis, functionalization and drug-loading of modular nano- and microsystems is completely innovative and the operative conditions to be chosen must be evaluate depending on the specific problem to be solved.

As an example, the following general considerations can be useful as a rule in the design of a modular nano- or microsystem:
1) Targeting molecule is linked to the chemical function through covalent or ionic bond.
2) Inclusion of the drug is obtained via adsorption in the pores of the material.
3) System must have a high affinity for the adsorption of guest molecule.
4) Release can be diffusive with slow profile or pH-controlled in environments in which pH is different from that of the medium where the system is administered.
5) Chemical functions can be obtained using 3-aminopropyltriethoxysilane reactants or any silicon alkoxide partially hydrolizable. They are present especially on both the external surface of the silica for steric hindrance reason and in the internal pores. In fact, in the case of the silica materials obtained through post-synthesis functionalization method, although the modification is carried out on materials with pores filled of surfactant, hybrid silica reaches the internal walls by migration of the reactant through micelles and further condensation on the walls.
6) Extraction process of the surfactant from the pore after functionalization must be compatible with the preservation of the biological activity of the targeting function. It can be carried out in water at room or slightly higher temperature, in a solvent which does not damage the targeting function or in a soxhlet extractor at moderate temperature. Drug-loading is possible even in complete or partial presence of surfactant, being the surfactant itself a dispersing agent and carrier. The choice of the elements constituting a modular system such as matrix, functionalization agent, targeting function and drug is one of the phases of the design of the system and is based on pharmacological, biological, physical and chemical considerations. In the design phase are also involved the knowledge of the adsorption kinetics of the drug in the solvent selected for drug-loading and of drug release from the system in the administration, crossed and release fluid.

### EXPERIMENTAL SECTION

### Examples of preparation of oxides and functionalized oxides with regular porosity.

### Process A1)

Preparation of a silica-micelles composite material.

The following reactants are employed: polyoxiethylene(10) isononylphenylether (Nonfix 10 Condea), sodium silicate (14% NaOH, 27% SiO₂; Aldrich), HCl 37% (Carlo Erba).

Sodium silicate is added to the surfactant solution after complete solubilization. Hydrocloric acid is added and the resulting gel is aged for 24 hours at room temperature and successively for further 24 hours at 100°C in a teflon-lined stainless-steel autoclave. The molar composition of the gel is:
SiO₂-0.78 NaOH-0.067 Nonfix10-0.82 HCl-58.9 H₂O

36.5 g of sodium silicate are added to the surfactant solution (7,25 g of Nonfix 10 in 143.5 g of H₂O) after complete dissolution of Nonfix 10. Lastly, 13.32 g of 37% HCl are added and the resulting gel is aged for 24 hours at room temperature and successively for further 24 hours at 100°C. The resulting suspension is filtered, washed and dried at 80°C.

### Process A2)

Preparation of a silica-micelles composite material.

The following reactants are employed: polyoxiethylene(10) isononilphenylether (Nonfix 10 Condea), tetraethylorthosilicate (Aldrich) and ethyl acetate (Aldrich). The molar composition of the gel is:
TEOS-4.30ethyl acetate-0.315Nonfix10-126.2H₂O
where TEOS indicates tetraethylorthosilicate.

13.77 g of Nonfix 10 are dissolved in 150 g of distilled water and 13.80 g of TEOS are dissolved in 25.0 g of ethyl acetate.

The organic phase which dissolves TEOS is slowly added to the water solution of surfactant. The system is aged at room temperature for 8 days under slow stirring. The organic phase is removed and the resulting suspension filtered, washed and dried at 80°C.

### Process A3)

Preparation of a silica-micelles composite material.

The following reactants are employed: polyoxiethylene(10) isononilphenylether (Nonfix 10 Condea) and tetraethylorthosilicate (Aldrich) and i-octane (Carlo Erba). The molar composition of the gel is:
TEOS-2.65i-octane-0.38Nonfix10-126.2H₂O.

16.74 g of Nonfix 10 are dissolved in 150 g of distilled water and 13.80 g of TEOS are dissolved in 20 g of i-octane.

The organic phase which dissolves TEOS is slowly added to the water solution of surfactant. The system is aged at room temperature for 8 days under slow stirring. The organic phase is removed and the resulting suspension filtered, washed and dried at 80°C.

### Process A4)

Preparation of a silica-micelles composite material.

The following reactants are employed: polyoxiethylene(10) isononilphenylether (Nonfix 10 Condea), tetraethylorthosilicate (Aldrich) and n-heptane (Aldrich). The molar composition of the gel is:
TEOS-2.65n-heptane-0.38Nonfix10-126.2H₂O

16.74 g of Nonfix 10 are dissolved in 150 g of distilled water and 13.78 g of TEOS are dissolved in 17.5 g of n-heptane.

The organic phase which dissolves TEOS is slowly added to the water solution of surfactant. The system is aged at room temperature for 8 days under slow stirring. The organic phase is removed and the resulting suspension filtered, washed and dried at 80°C.

### Process A5)

Preparation of a silica-micelles composite material.

The following reactants are employed: polyoxiethylene(10) isononilphenylether (Nonfix 10 Condea), tetraethylorthosilicate (Aldrich), i-octane (Carlo Erba) and ethylene glycol (Aldrich).

The molar composition of the gel is:
TEOS-2.65i-octane-0.38Nonfix10-25.6 H₂O-24.3ethylene glycol.

100.0 g of ethylene glycol and 16.7 g of (Nonfix 10 are dissolved in 30.5 g of distilled water and 13.80 g of TEOS are dissolved in 20.0 g of i-octane.

The organic phase which dissolves TEOS is slowly added to the water/ethylene glycol solution of surfactant. The system is aged at room temperature for 26 days under slow stirring. The organic phase is removed and the resulting suspension filtered, washed and dried at 80°C.

### Process B)

Preparation of a 3-aminopropyl mesoporous silica. Direct method. Sample AminoPropylSilicaDirect method (APSD).

The following reactants are employed: polyoxiethylene(10) isononylphenylether (Nonfix 10 Condea), sodium silicate (14% NaOH, 27% SiO₂; Aldrich), HCl 37% (Carlo Erba) and 3-aminopropyltriethoxysilane (Aldrich). The molar composition of the gel is:
TEOS-0.78NaOH-0.067Nonfix10-0.82HCl-0.042APTES-58.9H₂O
where APTES is 3-aminopropyltriethoxysilane.

36.5 g of sodium silicate are added to the surfactant solution (7,25 g of Nonfix 10 in 143.5 g of H₂O) after complete dissolution of Nonfix 10. Lastly, 13.32 g of 37% HCl and 45 g of APTES are added and the resulting gel is aged for 24 hours at room temperature and successively for further 24 hours at 100°C. The resulting suspension is filtered, washed and dried at 80°C.

### Process C)

Preparation of a 3-aminopropyl mesoporous silica. Direct method.

The following reactants are employed, polyoxiethylene(10) isononilphenylether(Nonfix 10 Condea), tetraethylorthosilicate (Aldrich) and 3-aminopropyltriethoxysilane (Aldrich).

The molar composition of the gel is:
TEOS-0.235 Nonfix10-0.061APTES-133,5 H₂O

The surfactant Nonfix 10 (9.8g, 0.0148 mol) is completely dissolved in 150 g of distilled water at room temperature. Successively TEOS (13.0 g, 0.0624 mol), and APTES (0.85 g, 0.0038 mol) are added. The mixture is aged under slow stirring for 8 days at room temperature. The resulting suspension is filtered washed and dried at 80°C.

### Process D)

Preparation of a 3-aminopropyl mesoporous silica. Post-synthesis functionalization of mesoporous silica. Sample AminoPropylSilicaPost-synthesis (APSP) 3 g of mesoporous silica obtained according process A1, A2, A3, A4, A5, are activated in oven for 2 hours at 120°C and then suspended in anhydrous toluene (25 ml). APTES (5 ml) is successively added and the suspension is kept under reflux for 7 hours. The solid is then filtered and washed with tetrahydrofuran.

### Examples of anchoring of folic acid on 3-aminopropyl mesoporous silica.

### Process E) Preparation of a APSD or APSP FOLic acid derivatized sample (APSD-FOL or APSP-FOL)

Triethylamine (0.25 ml) and folic acid (0.5 g) are dissolved in dimethylsulfoxide (15 ml). After complete dissolution, nitromethane (3 ml) and 3-aminopropyl mesoporous silica (36 g) obtained according to process B,C or D and previously activated in oven at 110°C for 2 hours, are added. Finally, 2.27 mmols of dicyclohexylcarbodiimide (0.47 g) or diisopropylcarbodiimide (0.29 g) are added. The mixtures is stirred for 30 hours in the dark.

Process F) Preparation of a APSD or APSP folic acid system.
Triethylamine (0.25 ml) and folic acid (0.5 g) are dissolved in dimethylsulfoxide (15 ml). After complete dissolution, nitromethane (3 ml) and 3-aminopropyl mesoporous silica (3.6 g) obtained according to process B,C or D and previously activated in oven at 110°C for 2 hours, are added. The mixtures is stirred for 30 hours in the dark.

### Example of surfactant extraction process

Process G) Surfactant extraction from APSD-FOL or APSP-FOL (Samples APSD-FOL-E or APSP-FOL-E).

Surfactant is removed by consecutives cycles of extraction using 3 grams of material in 1 liter of distilled water at 30 °C for 12 hours in order to preserve the biological activity of folic acid.

### Examples of drug-loading processes on folic acid-aminopropyl mesoporous silica.

Process H) Preparation of the system 3-aminopropyl mesoporous silica/folic acid /cisplatin. Samples APSD-FOL-E/cisplatin or APSP-FOL-E/cisplatin.

The impregnation of cisplatin in the folic acid/3-aminopropyl mesoporous silica is carried out as by a soaking process. 107 mg of material are suspended for 24 hours under continuos stirring at room temperature in a solution of cisplatin in water (38 mg in 45 ml). Successively, the solid is filtered, washed with 400 ml of bidistilled water during 2 hours and dried at 50°C.

Process I) Preparation of the system 3-aminopropyl mesoporous silica/folic acid/oxaliplatin. Samples APSD-FOL-E/oxaliplatin or APSP-FOL-E/oxaliplatin.

The impregnation of oxaliplatin in the3-aminopropyl mesoporous silica /folic acid after surfactant extraction is carried out as in process H. 100 mg of materials are suspended for 24 hours under continuos stirring at room temperature in a solution of oxaliplatin in water (5 mg in 6,3 ml). Successively the solid is filtered, washed with 400 ml of bidistilled water during 2 hours and dried at 50°C.

### RESULTS

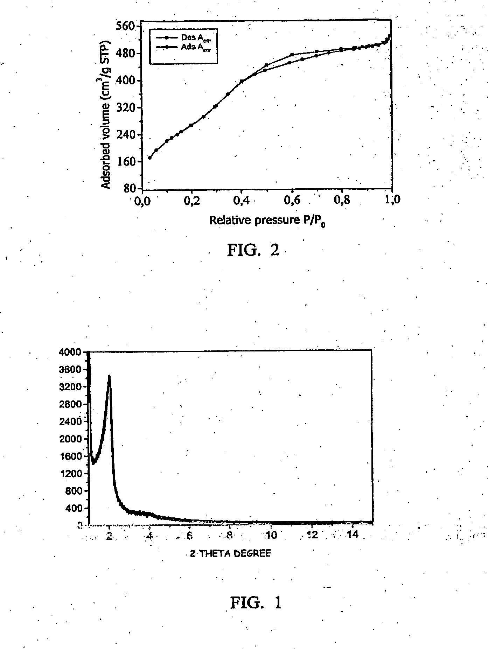

Synthesis process A allows to prepare ordered mesoporous materials which exhibit, after activation, specific surface area around 1000 m²/g and very high pore volume as shown by the powder XRD pattern (Figure 1) and nitrogen adsorption-desorption isotherms (Figure 2). The post-synthesis functionalization is carried out on the silica-micelles composite before surfactant removal so that the modifying agent is preferentially addressed on the external surface of the particle. The modifying agent can also migrate through the micelles and condense on the internal pore walls as demonstrated in the open literature for some kind of mesoporous silica.

Table 1 shows the results of elemental analysis on mesoporous 3-aminopropyl mesoporous silica materials obtained according to processes B (direct) and D (post-synthesis modification) indicated as APSD and APSP, respectively.

**Table 1**

| Sample | C (% by weight) | H (% by weight) | N (% by weight) |
|---|---|---|---|
| APSD | 24,06 | 4.10 | 1.01 |
| APSP | 11.00 | 3.02 | 2.99 |

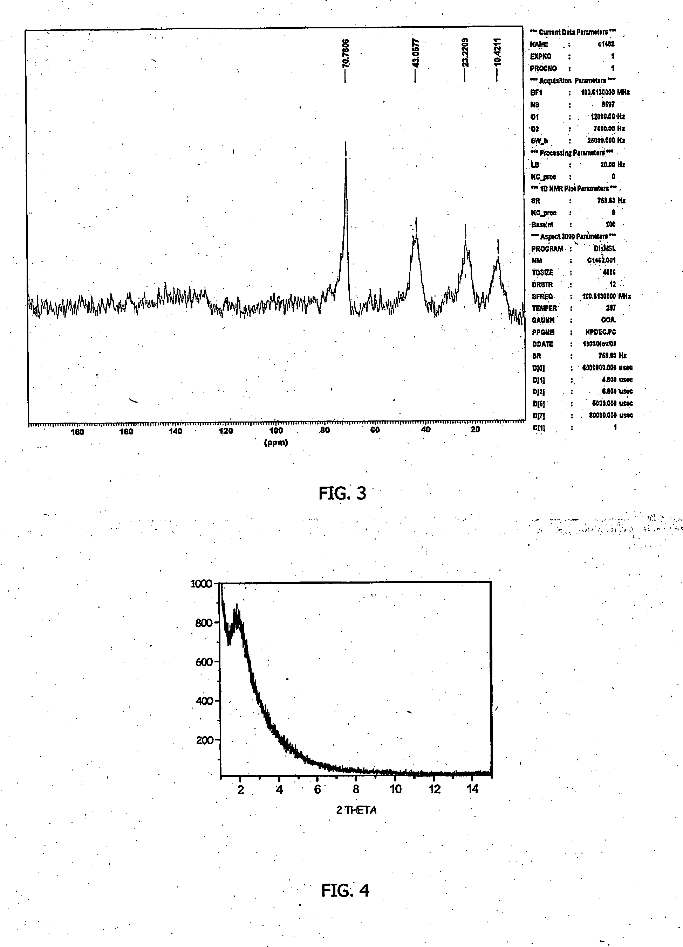

The presence of nitrogen in the materials confirms the covalent anchoring of aminopropyl groups and suggests that, in the case of post-synthesis process in toluene at 110°C, a partial surfactant extraction is possible. ¹³C NMR-MAS spectrum of sample APSP is shown in Figure 3.

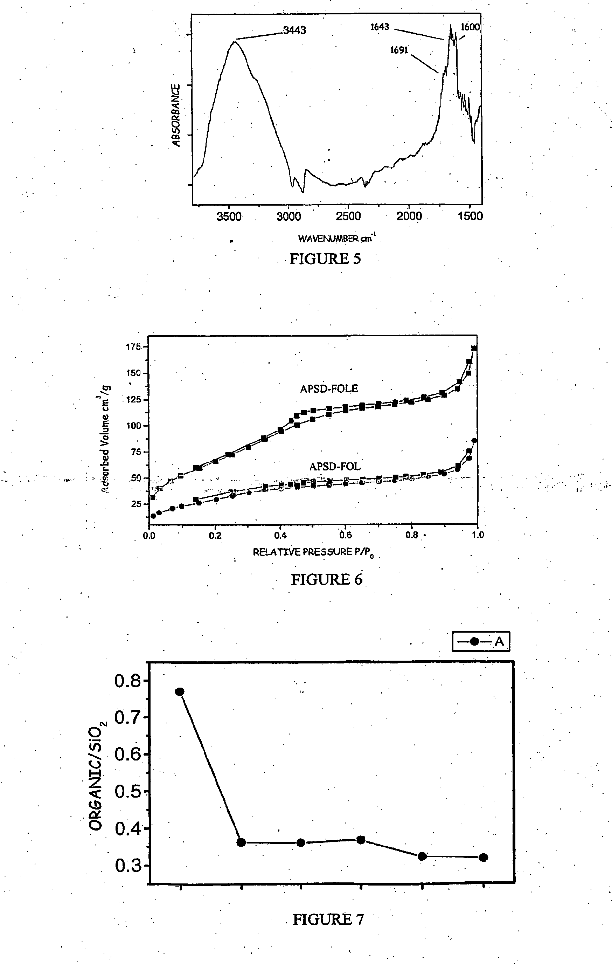

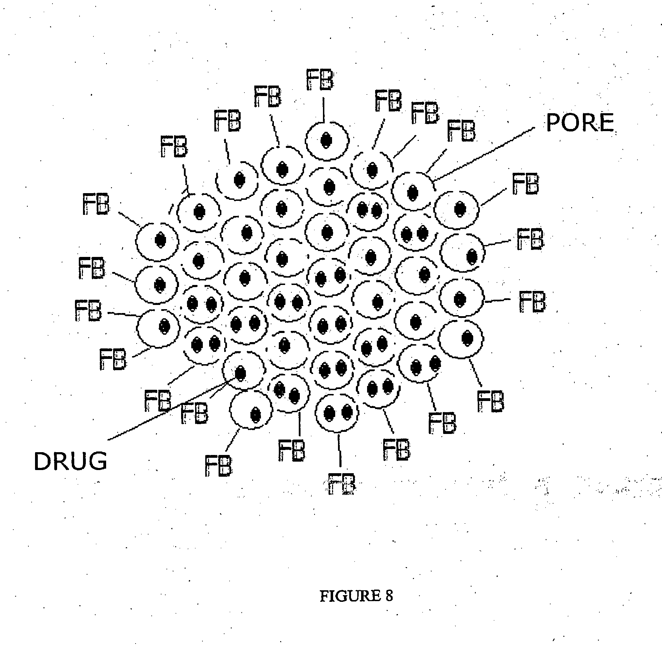

Figure 4 shows powder XRD diffraction pattern of a sample APSD-FOL-E obtained after covalent bonding of folic acid on the surface of a surfactant extracted 3-aminopropyl mesoporous silica (APSD-FOL). The single reflection in the XRD pattern (Figure 4) is maintained during functionalization and surfactant extraction processes. FT-IR spectrum of material APSD-FOL-E shows typical bands of folic acid molecule. Figure 6 shows nitrogen adsorption-desorption isotherms of sample APSD-FOL (bottom curve) and APSD-FOL-E (upper curve). Extraction process produces a remarkable pore volume with homogeneously-sized mesopores.

Table 2 shows elemental analysis data and modular composition of materials APSD-FOL-E and APSP-FOL-E (moles/silica moles). Modular composition is referred to the supramolecular system and in particular to the relative amounts of each unit constituting the hybrid system.

Figure 7 shows the variation of the organic/SiO₂ ratio as a function of the extraction processes and is referred to the APSD-FOL material.

**Table 2**

| Sample | (CH₂)₃-NH₂/SiO₂ molar ratio | Folic Acid/SiO₂ molar ratio | Nonfix 10/SiO₂ molar ratio |
|---|---|---|---|
| APSD-FOL-E | 0.0610 | 0.0138 | 0.0116 |
| APSP-FOL-E | 0.154 | 3.12·10⁻³ | 3.10·10⁻³ |

Synthesis process A2, A3, A4, A5 and C are developed in order to produce silica micelles composites with controlled morphology at the nanometer scale. Mesoporous structures at macroscopic scale are obtained in the interfacial region using water-oil systems at acid pH. Obtained mesostructures reproduce size and morphology of oil spheres because inorganic materials condense around them. Stirring represents a mechanism to control emulsion properties because it affects long range hydrodinamic forces. Slow stirring allows to obtain essentially fibrous morphology, while at increased stirring rate particles with spherical morphologies are produced. A biphasic two-level system is used to produce self-supporting membranes at the static interface between aqueous and organic phases [S. Schacht, Q. Huo, I.G. Voigt-Martin, G.D. Stucky, F. Schuth, Science, 273, (1996) 768].

More recently a mesoporous film has been prepared through the separation of the reagents in two separate phases, both in acid and in basic medium [L. Faget, A Bergman, O. Regev, Thin Solid Films, 386 (2001) 6].

In our experiments syntheses are carried out at room temperature and silica condensation occurs in the interfacial region between the lower water-surfactant solution and the upper organic phase containing the silica source which feeds the formation of the material.

In these conditions particle size is limited because the newly-formed particles move from the interfacial region and migrate by gravity to the bottom part of the synthesis vessel where silica is not present.

Drug loading of anticancer drug cisplatin and oxaliplatin (processes H and I) are carried out on two different amounts, respectively, of the material obtained by surfactant extraction process (APSD-FOL-E).

Tables 3 shows results obtained from elemental analysis of samples obtained by soaking processes H and I, (APSD-FOL-E/Cisplatin and APSD-FOL-E/Oxaliplatin, respectively).

**Table 3.**

| Sample | Pt /SiO₂ molar ratio |
|---|---|
| APSD-FOL-E/cisplatin | 0.00485 |
| APSD-FOL-E/Oxaliplatin | 0.0030 |

## Claims

1. Micro or nanosystems constituted of an inorganic oxide-based matrix with regular porosity controlled through the use of a generic surfactant or through the use of a mechanism of molecular imprinting, said micro or nanosystems being suitable for active targeting of molecules, atoms or ions with biological activity and being aimed at the local release in biological compartments of therapeutic interest, **characterized in that** the pores contain a biologically active substance, that a substance devoted to targeting and molecular recognition selected from folic acid, a peptide, an antibody, a glycoside, a carbohydrate or a protein is selectively coupled onto the external surface of said micro or nanosystem, and that the substance selectively coupled onto the external surface is not the biologically active substance contained within the pores.

2. Process for the preparation of a micro or nano-system made of an inorganic oxide-based matrix with regular porosity according to any previous claim wherein inorganic oxides are provided with a chemical function introduced during the synthesis of the micro or nano-system or through a post-synthesis grafting process on the external surface of the mesoporous micro or nano-system, wherein the targeting molecule is linked to the chemical function through covalent or ionic bond and wherein molecules, atoms or ions with biological activity are loaded in the pores of the micro or nano-system after the targeting molecule is linked to the chemical function and after removal of surfactant, **characterized in that** the anchoring process of folic acid as targeting substance, is produced with or without a condensing agent in the following way: - 0.25 ml of triethylamine and 0.5 g of folic acid are dissolved in 15 ml dimethylsulfoxide and, when the dissolution is complete, nitromethane and inorganic oxides previously activated in oven at 110°C for 2 hours are added, 2.27 mmoles of dicyclohexylcarbodiimide or diisopropylcarbodiimide is added or not and mixtures is finally stirred for 30 hours in the dark.

3. Process for the preparation of a micro or nano-system according to claim 2 wherein the inorganic oxide-based matrix is prepared using the following reactants: polyoxyethylene isononylphenylether, sodium silicate, HCl 37% and according to the following steps: Sodiumsilicate is added to a surfactant solution after complete solubilization; Hydrocloric acid is added and the resulting gel is aged for 24 hours at room temperature and successively for further 24 hours at 100°C in a teflon-lined stainless-steel autoclave, the resulting suspension is filtered, Washed and dried at 80°C.

4. Process for the preparation of a micro or nano-system according to claim 2 wherein an inorganic oxide provided with a chemical function introduced during the synthesis is prepared using the following reagents: polyoxyethylene isononylphenylether, tetraethylorthosilicate, 3-aminopropyltriethoxysilane and water and according to the following steps: a surfactant is dissolved in distilled water, successively tetraethylorthosilicate and 3-aminopropyltriethoxysilane are added, the mixture is aged under slow stirring for 8 days at room temperature, the resulting suspension is filtered washed and dried at 80°C.

5. Process for the preparation of a micro or nano-system according to claim 3 and 4 wherein the targeting molecule that is folic acid is linked, through covalent bond, to the chemical function, introduced during the synthesis or through a post-synthesis grafting process, of inorganic oxide using the following reagents: triethylamine, folic acid, dimethylsulfoxide, nitromethane, dicyclohexylcarbodiimide or diisopropylcarbodiimide, wherein triethylamine and folic acid are dissolved in dimethylsulfoxide, wherein, after complete dissolution, nitromethane and inorganic oxides previously activated in oven at 110°C for 2 hours, are added, and wherein finally, dicyclohexylcarbodiimide or diisopropylcarbodiimide is added, the mixtures is stirred for 30 hours in the dark.

6. Process for the preparation of a micro or nano-system according to claim 5 wherein the targeting molecule that is folic acid is linked, through ionic bond, to the chemical function, introduced during the synthesis or through a post-synthesis grafting process, of inorganic oxide using the following reagents: triethylamine, folic acid, dimethylsulfoxide, nitromethane, wherein triethylamine and folic acid are dissolved in dimethylsulfoxide, wherein after complete dissolution, nitromethane and inorganic oxides previously activated in oven at 110°C for 2 hours, are added, and wherein the mixtures is stirred for 30 hours in the dark.

7. Process for the preparation of a micro or nano-system according to claim 5 or 6 wherein surfactant is removed from inorganic oxides provided with a chemical function linked with the targeting molecule folic acid: surfactant is removed by consecutives cycles of extraction using 3 grams of material in 1 liter of distilled water at 30 °C for 12 hours in order to preserve the biological activity of folic acid.

8. Process for the preparation of a micro or nano-system according to claim / wherein cisplatin or oxaliplatin is loaded in the pores of the micro or nano-system after removal of surfactant: micro or nanosystems are suspended for 24 hours under continuous stirring at room temperature in a solution of cisplatin or oxaliplatin in water, successively, the solid is filtered, washed with bidistilled water, during 2 hours and dried at 50°C.

## Patentansprüche

1. Mikro- oder Nanosysteme, bestehend aus einer anorganischen Oxid-basierten Matrix mit regelmäßiger Pososität, die durch die Verwendung eines generischen oberflächenaktiven Mittels oder durch die Verwendung eines Mechanismus der molekularen Prägung gesteuert wird, wobei die Mikro- oder Nanosysteme für ein aktives Targeting von Molekülen, Atomen oder Ionen mit biologischer Aktivität geeignet sind und zur lokalen Freisetzung in biologischen Kompartimenten von therapeutischem Interesse vorgesehen sind, **dadurch gekennzeichnet, dass** die Poren eine biologisch aktive Substanz enthalten, dass eine Substanz, bestimmt für das Targeting und die molekulare Erkennung, ausgewählt aus Folsäure, einem Peptid, einem Antikörper, einem Glykosid, einem Kohlenhydrat oder einem Protein, selektiv an die äußere Oberfläche des Mikro- oder Nanosystems gekoppelt ist, und dass die Substanz, die selektiv auf die äußere Oberfläche gekoppelt ist, nicht die biologisch aktive Substanz ist, die in den Poren enthalten ist.

2. Verfahren zur Herstellung eines Mikro- oder Nanosystems aus einer anorganischen Oxid-basierten Matrix mit regelmäßiger Porosität nach einem der vorhergehenden Ansprüche, wobei anorganische Oxide mit einer chemischen Funktion bereitgestellt werden, die während der Synthese des Mikro- oder Nanosystems oder durch einen Pfropfprozess nach der Synthese auf der äußeren Oberfläche des mesoporösen Mikro- oder Nanosystems eingeführt werden, wobei das Targeting-Molekül an die chemische Funktion durch kovalente oder ionische Bindung gebunden wird, und wobei Moleküle, Atome oder Ionen mit biologischer Aktivität in die Poren des Mikro- oder Nanosystems geladen werden, nachdem das Targeting-Molekül an die chemische Funktion gebunden ist und nach der Entfernung von oberflächenaktivem Mittel, **dadurch gekennzeichnet, dass** der Verankerungsprozess von Folsäure als Targeting-Substanz mit oder ohne ein Kondensationsmittel auf folgende Weise hergestellt wird:
- 0,25 ml Triethylamin und 0,5 g Folsäure werden in 15 ml Dimethylsulfoxid gelöst, und wenn die Lösung beendet ist, werden Nitromethan und anorganische Oxide, die zuvor im Ofen bei 110 °C während 2 Stunden aktiviert wurden, zugegeben, 2,27 Mol Dicyclohexylcarbodiimid oder Diisopropylcarbodiimid werden zugegeben oder nicht, und die Mischungen werden schließlich während 30 Stunden im Dunkeln gerührt.

3. Verfahren zur Herstellung eines Mikro- oder Nanosystems nach Anspruch 2, wobei die anorganische Oxid-basierte Matrix unter Verwendung der folgenden Reaktanten hergestellt wird: Polyoxyethylenisononylphenylether, Natriumsilikat, HCl 37 % und gemäß den folgenden Schritten: Natriumsilikat wird einer oberflächenaktiven Lösung nach der vollständigen Solubilisierung zugegeben; Chlorwassertoffsäure wird zugegeben, und das sich ergebende Gel wird während 24 Stunden bei Raumtemperatur und dann während weiteren 24 Stunden bei 100 °C in einem Teflon-verkleideten Edelstahl-Autoklaven gealtert, die erhaltene Suspension wird gefiltert, gewaschen und bei 80 °C getrocknet.

4. Verfahren zur Herstellung eines Mikro- oder Nanosystems nach Anspruch 2, wobei ein anorganisches Oxid, bereitgestellt mit einer chemischen Funktion, eingeführt während der Synthese, unter Verwendung der folgenden Reagenzien hergestellt wird: Polyoxyethylenisononylphenylether,
Tetraethylorthosilicat, 3-Aminopropyltriethoxysilan und Wasser und gemäß den folgenden Schritten: ein oberflächenaktives Mittel wird in destilliertem Wasser gelöst, dann werden Tetraethylorthosilicat und 3-Aminopropyltriethoxysilan zugegeben, die Mischung wird unter langsamem Rühren während 8 Tagen bei Raumtemperatur gealtert, die sich ergebende Suspension wird gefiltert, gewaschen und bei 80 °C getrocknet.

5. Verfahren zur Herstellung eines Mikro- oder Nanosystems nach Anspruch 3 und 4, wobei das Targeting-Molekül, bei dem es sich um Folsäure handelt, durch ein kovalente Bindung an die chemische Funktion, eingeführt während der Synthese oder durch einen Pfropfprozess nach der Synthese, von anorganischem Oxid unter Verwendung der folgenden Reagenzien gebunden wird: Triethylamin, Folsäure, Dimethylsulfoxid, Nitromethan, Dicyclohexylcarbodiimid oder Diisopropylcarbodiimid, wobei Triethylamin und Folsäure in Dimethylsulfoxid gelöst werden, wobei, nach der vollständigen Lösung, Nitromethan und anorganische Oxide, die zuvor im Ofen bei 110 °C während 2 Stunden aktiviert wurden, zugegeben werden, und wobei schließlich Dicyclohexylcarbodiimid oder Diisopropylcarbodiimid zugegeben wird, wobei die Mischung während 30 Stunden im Dunkeln gerührt wird.

6. Verfahren zur Herstellung eines Mikro- oder Nanosystems nach Anspruch 5, wobei das Targeting-Molekul, bei dem es sich um Folsäure handelt, durch eine ionische Bindung an die chemische Funktion, eingeführt während der Synthese oder durch einen Pfropfprozess nach der Synthese, von anorganischem Oxid unter Verwendung der folgenden Reagenzien gebunden wird: Triethylamin, Folsäure, Dimethylsulfoxid, Nitromethan, wobei Triethylamin und Folsäure in Dimethylsulfoxid gelöst werden, wobei nach der vollständigen Lösung Nitromethan und anorganische Oxide, die zuvor im Ofen bei 110 °C während 2 Stunden aktiviert wurden, zugegeben werden, und wobei die Mischung während 30 Stunden im Dunkeln gerührt wird.

7. Verfahren zur Herstellung eines Mikro- oder Nanosystems nach Anspruch 5 oder 6, wobei oberflächenaktives Mittel von anorganischen Oxiden entfernt wird, die mit einer chemischen Funktion bereitstellt werden, die mit dem Targeting-Molekül Folsäure verbunden ist: oberflächenaktives Mittel wird durch aufeinander folgende Extraktionszyklen unter Verwendung von 3 Gramm Material in 1 Liter destilliertem Wasser bei 30 °C während 12 Stunden entfernt, um die biologische Aktivität von Folsäure beizubehalten.

8. Verfahren zur Herstellung eines Mikro- oder Nanosystems nach Anspruch 7, wobei Cisplatin oder Oxaliplatin in die Poren des Mikro- oder Nanosystems nach der Entfernung des oberflächenaktiven Mittels geladen wird: Mikro- oder Nanosysteme werden während 24 Stunden unter kontinuierlichem Rühren bei Raumtemperatur in einer Lösung von Cisplatin oder Oxalipatin in Wasser suspendiert, dann wird der Feststoff gefiltert, mit bidestilliertem Wasser während zwei Stunden gewaschen und bei 50 °C getrocknet.

## Revendications

1. Micro ou nanosystèmes constitués d'une matrice inorganique à base d'oxyde ayant une porosité régulière contrôlée par l'utilisation d'un agent tensioactif générique ou par l'utilisation d'un mécanisme d'impression moléculaire, lesdits micro ou nanosystèmes étant appropriés pour le ciblage actif moléculaire, des atomes ou des ions avec une activité biologique et étant destinés à la libération locale dans des compartiments biologiques d'intérêt thérapeutique, **caractérisés en ce que** les pores contiennent une substance biologiquement active, **en ce que** une substance consacrée au ciblage et à la reconnaissance moléculaire choisie parmi l'acide folique, un peptide, un anticorps, un glycoside, un glucide ou une protéine est couplée de manière sélective sur la surface extérieure dudit micro ou nanosystème, et **en ce que** la substance couplée de manière sélective sur la surface extérieure n'est pas la substance biologiquement active contenue dans les pores.

2. Procédé pour la préparation d'un micro ou nanosystème constitué d'une matrice inorganique à base d'oxyde ayant une porosité régulière selon l'une quelconque des revendications précédentes, où les oxydes inorganiques sont pourvus d'une fonction chimique introduite au cours de la synthèse des micro ou nanosystème ou à travers un procédé de greffage post-synthèse sur la surface extérieure du micro ou nanosystème mésoporeux, où la molécule de ciblage est liée à la fonction chimique à travers une liaison covalente ou ionique et où des molécules, des atomes ou des ions ayant une activité biologique sont chargés dans les pores du micro ou nanosystème après la liaison de la molécule de ciblage à la fonction chimique et après l'enlèvement du tensioactif, **caractérisé en ce que** le procédé d'ancrage d'acide folique en tant que substance de ciblage, est produit avec ou sans un agent de condensation de la manière suivante :
- dissoudre 0,25 ml de triéthylamine et 0,5 g d'acide folique dans 15 ml de diméthylsulfoxyde et, au terme de la dissolution, ajouter nitrométhane et des oxydes inorganiques préalablement activés dans un four à 110°C pendant 2 heures, ajouter ou ne pas ajouter 2,27 moles de dicyclohexylcarbodiimide ou de diisopropylcarbodiimide et, enfin, agiter les mélanges pendant 30 heures dans l'obscurité.

3. Procédé pour la préparation d'un micro ou nanosystème selon la revendication 2, où la matrice inorganique à base d'oxyde est préparée en utilisant les réactants suivants : polyoxyéthylène isononylphenyléther, silicate de sodium, HCl 37% et selon les étapes suivantes : ajouter le sodiumsilicate à une solution tensioactive après solubilisation complète ; ajouter acide chlorhydrique et vieillir le gel résultant pendant 24 heures à température ambiante et successivement encore pendant 24 heures à 100°C dans un autoclave en acier inoxydable recouvert de téflon, filtrer, laver et sécher à 80°C la suspension résultante.

4. Procédé pour la préparation d'un micro ou nanosystème selon la revendication 2, où un oxyde inorganique pourvu d'une fonction chimique introduite au cours de la synthèse est préparé en utilisant les réactifs suivants polyoxyéthylène isononylphenyléther, tétraéthylorthosilicate, 3-aminopropyltriéthoxysilane et de l'eau, et selon les étapes suivantes : dissoudre un tensioactif dans de l'eau distillée, successivement ajouter tétraéthylorthosilicate et 3-aminopropyltriéthoxysilane, vieillir le mélange sous agitation lente pendant 8 jours à température ambiante, filtrer, laver et sécher à 80°C la suspension résultante.

5. Procédé pour la préparation d'un micro ou nanosystème selon la revendication 3 et 4, où la molécule de ciblage qui est acide folique est liée, à travers une liaison covalente, à la fonction chimique, introduite au cours de la synthèse ou à travers un procédé de greffage post-synthèse, d'oxyde inorganique en utilisant le réactifs suivants : triéthylamine, acide folique, diméthylsulfoxyde, nitrométhane, dicyclohexylcarbodiimide ou diisopropylcarbodiimide, où on a dissous triéthylamine et acide folique dans diméthylsulfoxyde, où, après dissolution complète, on a ajouté nitrométhane et des oxydes inorganiques préalablement activés dans un four à 110°C pendant 2 heures et où, enfin, on a ajouté dicyclohexylcarbodiimide ou diisopropylcarbodiimide, on a agité les mélanges pendant 30 heures dans l'obscurité.

6. Procédé pour la préparation d'un micro ou nanosystème selon la revendication 5 où la molécule de ciblage qui est acide folique est liée, à travers une liaison ionique, à la fonction chimique, introduite au cours de la synthèse ou à travers un procédé de greffage post-synthèse, d'oxyde inorganique en utilisant le réactifs suivants : triéthylamine, acide folique, diméthylsulfoxyde, nitrométhane, où on a dissous triéthylamine et acide folique dans diméthylsulfoxyde, où, après dissolution complète, on a ajouté nitrométhane et des oxydes inorganiques préalablement activés dans un four à 110°C pendant 2 heures et où on a agité les mélanges pendant 30 heures dans l'obscurité.

7. Procédé pour la préparation d'un micro ou nanosystème selon la revendication 5 ou 6 où le tensioactif est enlevé à partir des oxydes inorganiques pourvus d'une fonction chimique liée à la molécule de ciblage qui est acide folique : enlever le tensioactif par des cycles consécutifs d'extraction en utilisant 3 grammes de matériau dans 1 litre d'eau distillée à 30°C pendant 12 heures, afin de préserver l'activité biologique de l'acide folique.

8. Procédé pour la préparation d'un micro ou nanosystème selon la revendication 7, où on a chargé cisplatine ou oxaliplatine dans les pores du micro ou nanosystème après l'enlèvement du tensioactif : mettre en suspension les micro ou nanosystème pendant 24 heures sous conditions d'agitation à température ambiante dans une solution de cisplatine ou d'oxaliplatine dans l'eau, successivement, filtrer, laver deux fois avec eau distillée pendant 2 heures e sécher a 50°C le solide.
